# EUROPEAN PATENT APPLICATION

(11) **EP 2 810 684 A1**
(43) Date of publication of application: **10.12.2014**
(21) Application number: 12867650.9
(22) Date of filing: 16.11.2012
(51) Int. Cl.: A61M 25/14, A61M 25/10

(54) **HUB, AND CATHETER WITH HUB**

(30) Priority: 03.02.2012 JP 2012022332
(71) Applicant: Terumo Kabushiki Kaisha, Tokyo 151-0072 (JP)
(72) Inventor: SAWADA, Akira, Fujinomiya-shi Shizuoka 418-0015 (JP)
(74) Representative: Casalonga
(86) International application number: PCT/JP2012/079767
(87) International publication number: WO 2013/114706

(57) **Abstract**

A hub (10) is connected to a shaft (14) which includes a first lumen (18) extending in an axial direction and capable of allowing inserting of a guide wire (26) and a second lumen (22) extending in parallel with the first lumen (18). The hub (10) includes a holding unit (45) which holds the shaft (14), a distribution path (50) which communicates with the first lumen (18), and is capable of allowing inserting of the guide wire (26) and distributing a predetermined fluid, and a branch path (52) which communicates the second lumen (22) and the distribution path (50), is capable of distributing the predetermined fluid, and blocks the insertion of the guide wire (26).

## Description

### Technical Field

The present invention relates to a hub connected to a shaft which can be inserted into a living body and to a catheter having this hub.

### Background Art

In general, in a catheter used for medical use, a hub is connected to a proximal side of a shaft such that an operator can easily operate the shaft when inserting, entering, and retreating the shaft into or from a living body.

For example, a balloon catheter disclosed in JP-A-2006-262932 includes a catheter tube (shaft) having a dual structure (dual tube) with an inner tube (first shaft) and an outer shaft (second shaft), and a connector (hub) is connected to a proximal side of the shaft. This connector is provided with a first connector port communicating with a lumen of the first shaft, and a second connector port communicating with a lumen of the second shaft. In addition, a guide wire can be inserted into the first shaft through the first connector port.

### Summary of Invention

When a catheter is used, a priming operation (also referred to as flashing) of filling a shaft (lumen) with a fluid (for example, a physiological salt solution) before insertion into a living body is performed to mainly remove the air inside.

In a case where the priming operation in the catheter disclosed in JP-A-2006-262932 is performed, a syringe is connected to a first connector port and a fluid is supplied to a lumen of a first shaft. Further, the syringe is connected to a second connector and the fluid is supplied to the lumen of the second shaft. That is, time and labor are required for the priming operation to connect a syringe and supply a fluid for each port.

Here, a configuration in which a shared input port communicating with both lumens of the first and second shafts is provided in the hub and a liquid is supplied thereto from the shared input port is considered. However, in such a configuration, when a guide wire is inserted into the first shaft, the guide wire may wrongly enter the second shaft without entering the first shaft because the guide wire is inserted thereto through the shared input port, so there is a possibility that the guide wire may become deformed (or damaged).

The present invention has been made in light of the above problem and relates to a hub which efficiently supplies a fluid by allowing the fluid to simultaneously flow into a plurality of lumens, and prevents damage or deformation of a wire by inhibiting the wire wrongly entering a lumen other than lumens for wire insertion among a plurality of lumens and to a catheter having this hub.

For achieving the above-described object, the present invention provides a hub which is connected to a shaft including a lumen in which a wire is capable of being inserted, and a cavity portion separately provided from the lumen, including: a holding unit which holds the shaft; a distribution path which communicates with the lumen of the shaft held by the holding unit and is capable of allowing inserting of the wire and distributing a fluid; and a branch path which communicates the cavity portion of the shaft held by the holding unit with the distribution path, is capable of distributing the fluid, and blocks the insertion of the wire.

According to the above description, since the branch path communicates the cavity portion with the distribution path and the distribution of the fluid is possible, the fluid supplied to the distribution path is distributed through the lumen and can be allowed to be distributed through the cavity portion through the branch path. Accordingly, the fluid can be supplied to the lumen and the cavity portion at the same time, so a supplying operation (for example, priming operation or the like) of the fluid can be efficiently performed. Further, since the branch path blocks the entry of the wire, the wire is inhibited from wrongly entering the cavity portion. As a result, an inserting operation of the wire can be performed in a short period of time while preventing deformation or damage of the wire.

Here, the hub is configured to be connected to the shaft which includes a first shaft having the lumen and a second shaft having the cavity portion and capable of accommodating the first shaft in the cavity portion, the holding unit includes a first holding unit which holds the first shaft and a second holding unit which is provided on a more distal side than the first holding unit and holds the second shaft, the distribution path is provided on the proximal side of the first holding unit, and the branch path includes a side path which communicates with the distribution path and extends in the axial direction on the lateral side of the first holding unit.

In this way, in the hub which can be connected to the shaft (multiple tubes) accommodating the first shaft in the cavity portion of the second shaft, since the side path communicates with the distribution path and extends in the axial direction on the lateral side of the first holding unit, the fluid supplied to the distribution path can easily flow into the cavity portion by passing through the lateral side of the first holding unit. That is, the priming operation can be easily performed even with the shaft formed of the multiple tubes.

Further, the side path may be configured such that an opening portion opened to the distribution path is formed in a shape capable of blocking the entry of the distal portion of the wire.

In this way, since the opening portion of the side path is formed in a shape for blocking the entry of the distal portion of the wire, the entry of the distal portion of the wire can be inhibited by the opening portion of the side path. Therefore, the distal portion of the wire can be directed toward the lumen which communicates with the distribution path.

In this case, it is preferable that a plurality of the side paths be provided along the circumferential direction of the first holding unit.

Since the fluid can be distributed through the plurality of side paths, a sufficient amount of the fluid can be allowed to flow into the cavity portion.

Further, a groove portion capable of leading the fluid into the side path is provided on the inner surface constituting the distribution path.

In this way, the groove portion is provided on the inner surface constituting the distribution path, so the fluid supplied to the distribution path can be smoothly guided into the side path.

Further, the hub may be configured by a first housing and a second housing which are separated from each other in the axial direction.

In this way, the first and second housings of the hub can be separated from each other, so the inside of the hub can be accessed from the separated portion and cleaning the inner portion of the hub or the like can be easily performed.

Further, the hub may be configured such that the first housing and the second housing which are separated from each other are rotatably connected to each other, and the branch path may be configured such that the communication between the cavity portion and the distribution path is blocked along with relative rotation of the first housing with respect to the second housing.

In this way, the communication between the cavity portion and the distribution path is blocked along with the relative rotation of the first housing with respect to the second housing, so the fluid with respect to the cavity portion can be selectively supplied. For example, in a case where a contrast agent (fluid) is supplied when a shaft is inserted into a living body, the contrast agent can be supplied only to the lumen without being supplied to the cavity portion. Therefore, the contrast agent is ejected only from the distal side communicating with the lumen and the distal side of the shaft can be read with high precision.

Further, for achieving the above-described object of the present invention, a catheter includes the hub having the above configuration and the shaft, in which a space portion capable of accommodating an implant implanted in a living body is provided on an outer surface of the distal side of the first shaft, and the second shaft is capable of supplying the fluid which is guided into the distal side through the cavity portion to the space portion.

According to the above description, in the catheter delivering the implant, the fluid supplied to the distribution path can be distributed through not only the lumen but also the cavity portion through the branch path by the branch path communicating the cavity portion with the distribution path. Accordingly, the fluid can be supplied to the lumen and the cavity portion at the same time, so the priming operation or the like can be efficiently performed. In addition, the fluid ejected from the second shaft can be supplied to the implant by the priming operation. Moreover, since the branch path blocks the entry of the wire, the wire is inhibited from wrongly entering the cavity portion. As a result, deformation or damage of the wire is prevented and the insertion operation of the wire can be performed in a short period of time.

In this case, it is preferable that the second shaft includes an ejection hole which communicates with the space portion and ejects the fluid.

In this way, the second shaft includes an ejection hole which communicates with the space portion, so the fluid can be easily supplied to the implant accommodated in the space portion.

According to the present invention, the supplying operation of the fluid becomes efficient because it is possible for the fluid to flow into the plurality of lumens at the same time, and deformation or damage of the wire can be prevented by inhibiting the wire from wrongly entering the lumen other than the lumens for wire insertion among the plurality of lumens.

### Brief Description of Drawings

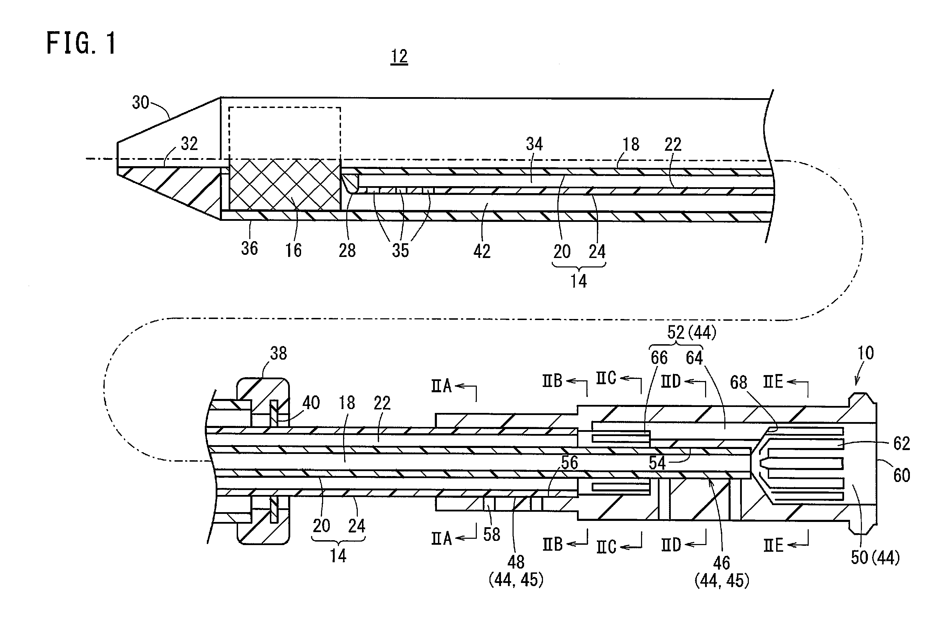

[Fig. 1] Fig. 1 is a partial cross-sectional side view illustrating the entire configuration of a catheter which includes a hub according to a first embodiment.

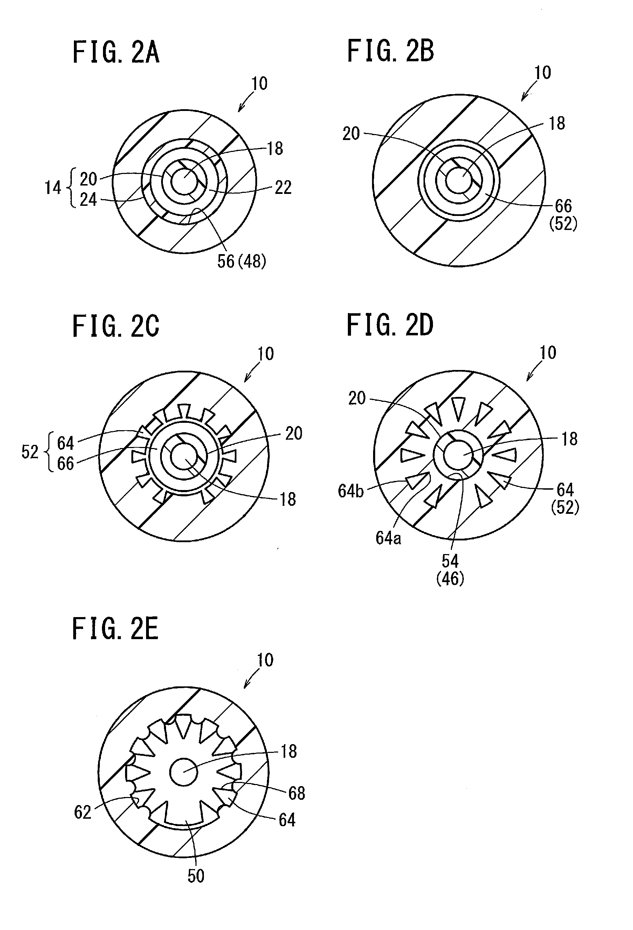

[Fig. 2] Fig. 2A is a cross-sectional view taken along line IIA-IIA of the hub of Fig. 1, Fig. 2B is a cross-sectional view taken along line IIB-IIB of Fig. 1, Fig. 2C is a cross sectional view taken along line IIC-IIC of Fig. 1, Fig. 2D is a cross-sectional view taken along line IID-IID of Fig. 1, and Fig. 2E is a cross-sectional view taken along line IIE-IIE of Fig. 1.

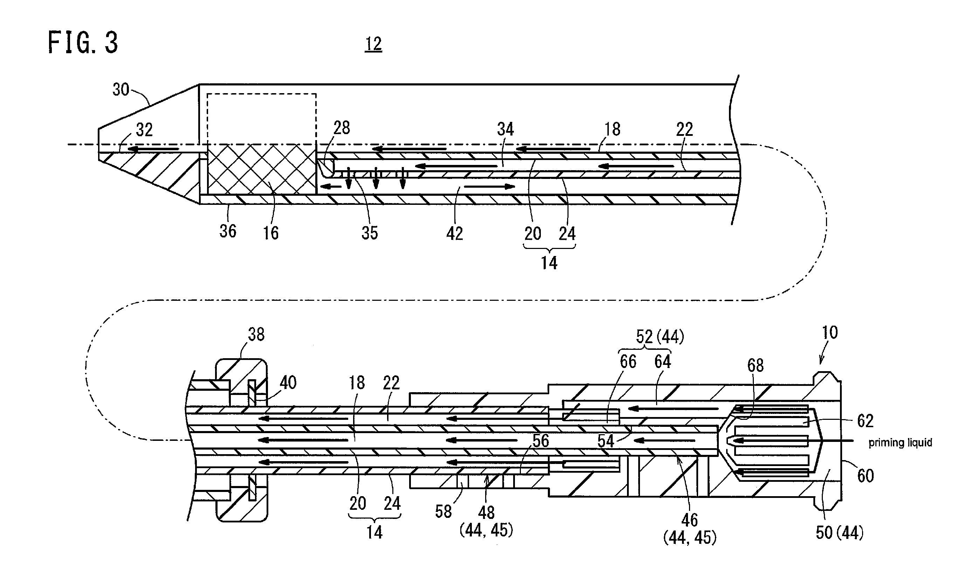

[Fig. 3] Fig. 3 is a partial cross-sectional side view illustrating flow of a fluid at the time of a priming operation in the catheter of Fig. 1.

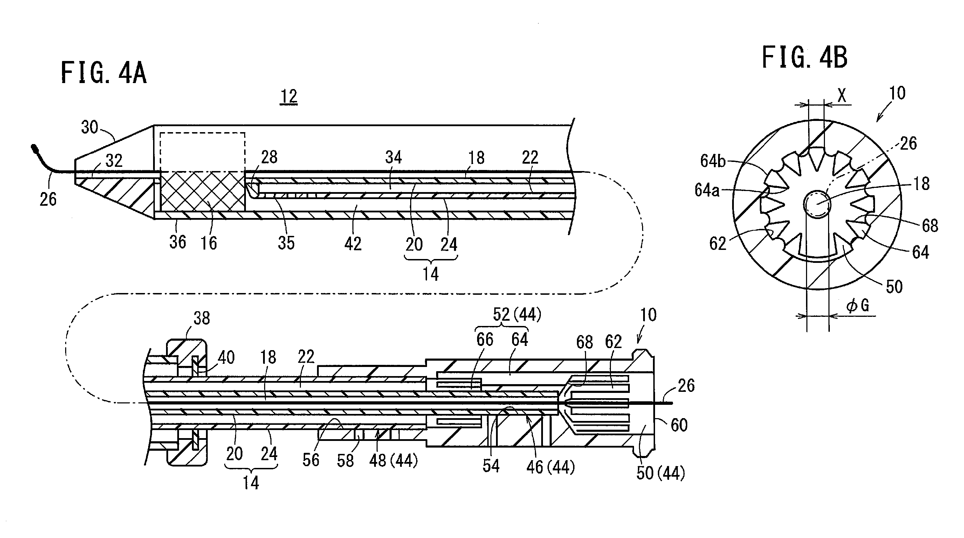

[Fig. 4] Fig. 4A is a partial cross-sectional side view illustrating a state at the time of inserting a guide wire in the catheter of Fig. 1, and Fig. 4B is an explanatory view illustrating a relationship between a proximal end communicating port of a side path and the guide wire.

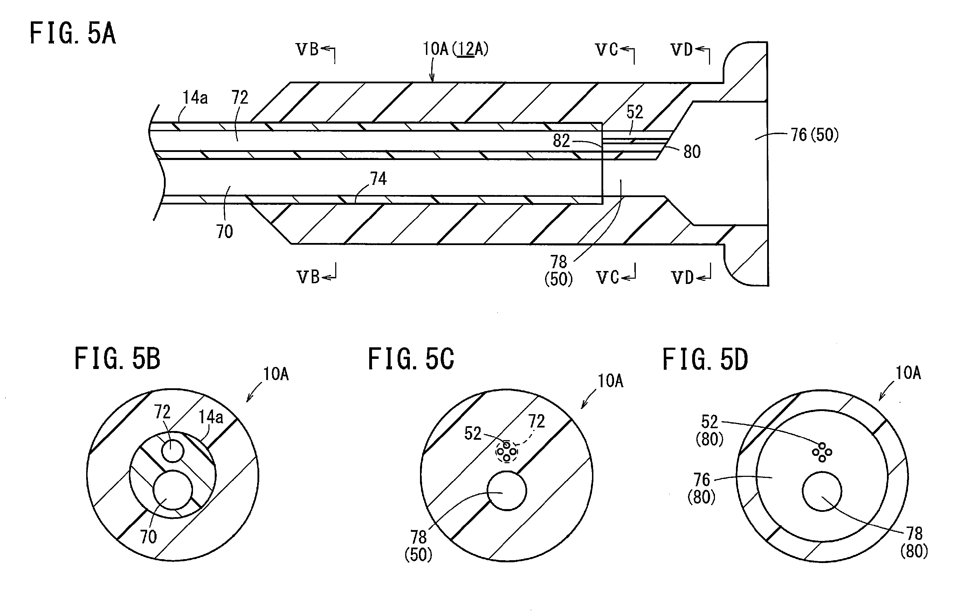

[Fig. 5] Fig. 5A is a partial cross-sectional view illustrating a hub of a catheter according to a first modification example, Fig. 5B is a cross-sectional view taken along line VB-VB of Fig. 5A, Fig. 5C is a cross-sectional view taken along line VC-VC of Fig. 5A, and Fig. 5D is a cross-sectional view taken along line VD-VD of Fig. 5A.

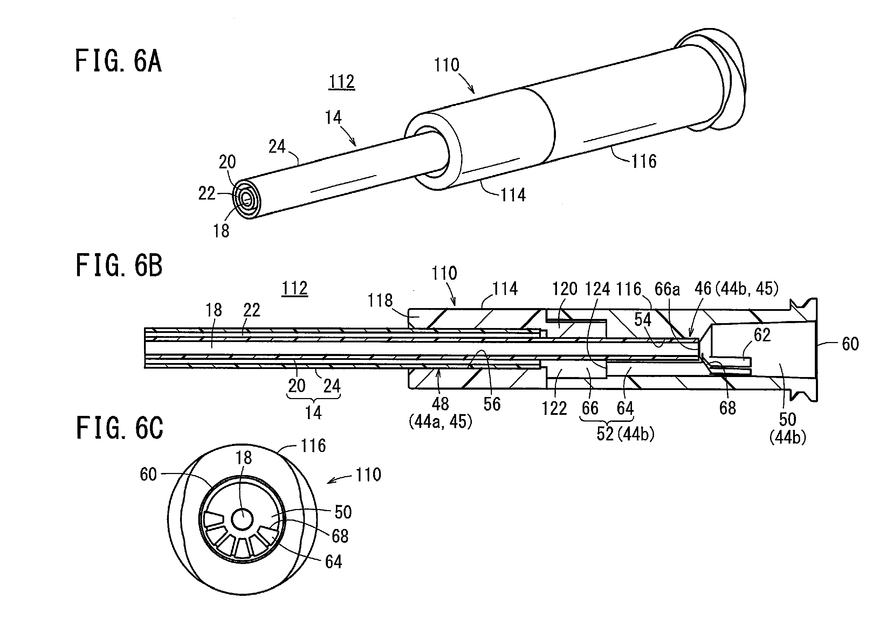

[Fig. 6] Fig. 6A is a perspective view illustrating a main part of a hub of a catheter according to a second embodiment, Fig. 6B is a cross-sectional side view of the hub of Fig. 6A, and Fig. 6C is an explanatory view illustrating the hub of Fig. 6A when being visually checked from a proximal end direction.

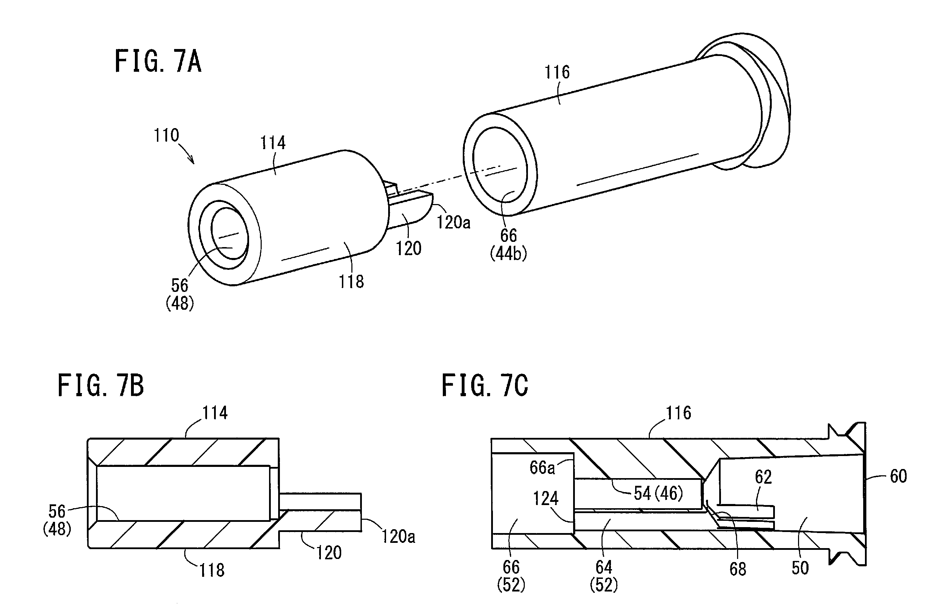

[Fig. 7] Fig. 7A is an exploded perspective view of the hub of Fig. 6A, Fig. 7B is a cross-sectional side view of a first housing of Fig. 7A, and Fig. 7C is a cross-sectional side view of a second housing of Fig. 7A.

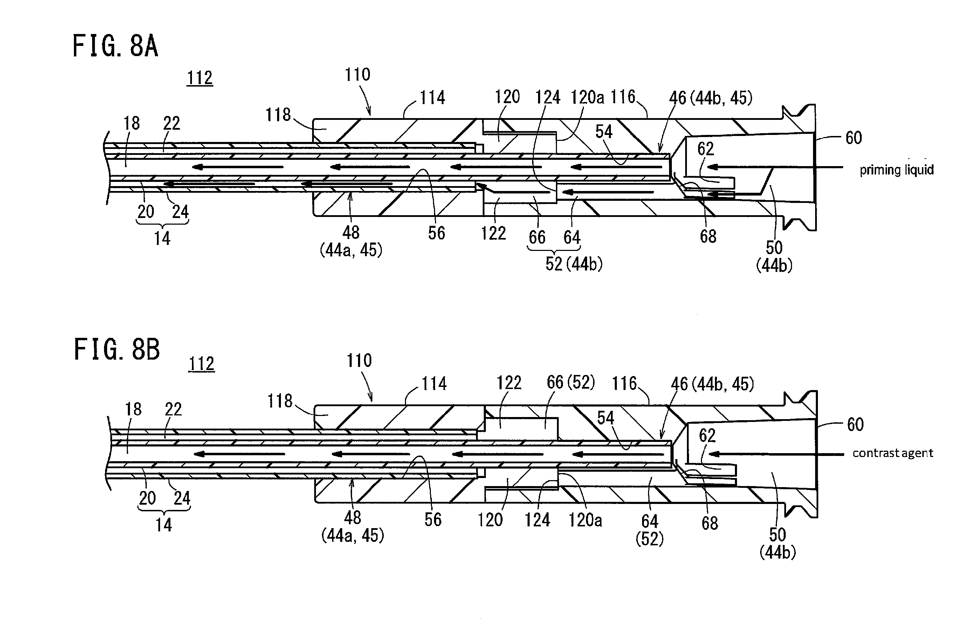

[Fig. 8] Fig. 8A is a partial cross-sectional side view illustrating flow of a fluid supplied to the hub of Fig. 6A, and Fig. 8B is a partial cross-sectional side view illustrating flow of a fluid in a state in which a first housing and a second housing of the hub of Fig. 8A are relatively rotated by 180° with respect to each other.

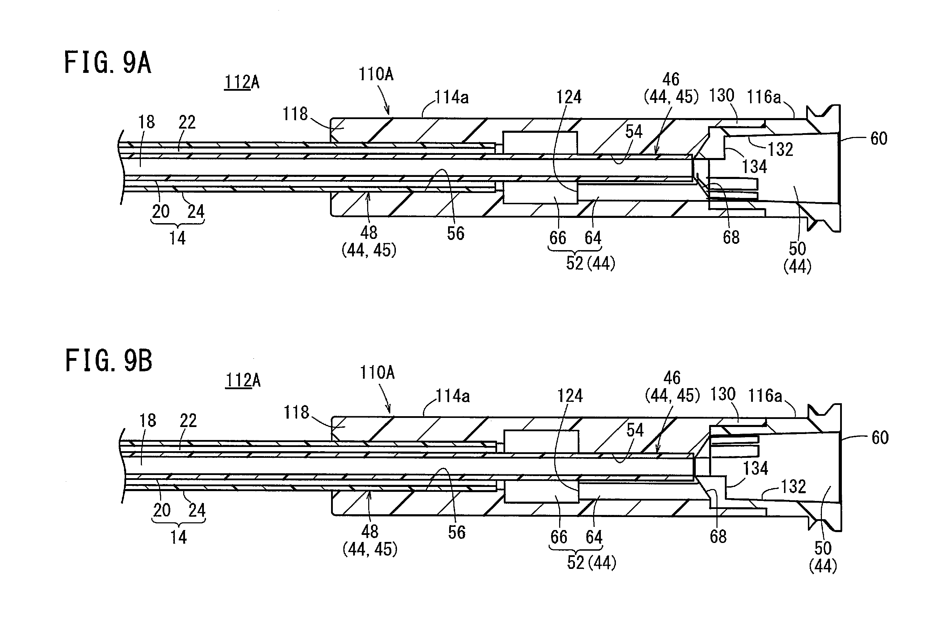

[Fig. 9] Fig. 9A is a partial cross-sectional view illustrating a hub of a catheter according to a second modification example, and Fig. 9B is a partial cross-sectional side view illustrating a state in which a first housing and a second housing of the hub of Fig. 9A are relatively rotated by 180° with respect to each other.

### Description of Embodiments

Hereinafter, a hub according to the present invention will be specifically described by the examples of preferred embodiments (a first embodiment and a second embodiment) in regard to the relationship between the hub and a catheter having the hub and with reference to the accompanying drawings.

### [First embodiment]

Fig. 1 is a partial cross-sectional side view illustrating the entire configuration of a catheter 12 which includes a hub 10 according to a first embodiment. The catheter 12 according to the first embodiment is configured as a delivery device which delivers/indwells an implant to/in a living body. The catheter 12 includes a shaft 14 having two lumens (dual lumen) and a hub 10 connected to a proximal side of the shaft 14. The catheter 12 is delivered into a living body lumen (for example, blood vessel) by an operator and the implant held at a distal side of the shaft 14 is guided to a desired site in a living body. As this implant, for example, a stent graft 16 which is accommodated in the catheter 12 in a contracted state and indwelled in a living body lumen by being expanded is exemplified.

The shaft 14 is formed of an elongate tube having flexibility and configured as multiple tubes (dual tube) in which two shafts having different diameters from each other are disposed on the same axis. Specifically, the shaft 14 is configured by an inner shaft 20 (first shaft) which includes a first lumen 18 extending along the axial direction and an outer shaft 24 (second shaft) which includes a second lumen 22 (cavity portion) extending along the axial direction in the same manner, and the inner shaft 20 is disposed in the outer shaft 24 (second lumen 22). Due to this, the inner surface of the outer shaft 24 constructing the second lumen 22 is formed such that the internal diameter thereof is sufficient for inserting the inner shaft 20. On the other hand, the inner surface of the inner shaft 20 constituting the first lumen 18 is formed such that the internal diameter thereof is sufficient for inserting a preceding guide wire 26 (see Fig. 4A) at the time of delivering the catheter 12.

In other words, the catheter 12 according to the first embodiment is an over-the-wire type of catheter using the first lumen 18 for inserting a guide wire, which is disposed inside among the dually-formed lumens. On the other hand, in regard to the second lumen 22, there are various methods of using the second lumen according to an implant or the like to be delivered. For example, in a case where a balloon-expanding type of stent graft is adopted as an implant, the stent graft is used for a flow path which supplies a fluid to a balloon expanding the stent graft. In the present embodiment, a self-expanding type of stent graft 16 is adopted as an implant, and the distal side of the second lumen 22 is sealed by a first sealing member 28.

The inner shaft 20 functions as a partition wall interposed between the first lumen 18 extending along the inside thereof and the second lumen 22 extending along the outside thereof. In addition, the guide wire 26 inserted into the first lumen 18 can easily follow the inner shaft 20 which extends in the vicinity of the axial center of the catheter 12.

A nose cone 30 whose side surface is formed as a tapered surface is connected to the distal portion of the inner shaft 20. An insertion path 32 communicating with the first lumen 18 is formed inside of the nose cone 30. The nose cone 30 sends out the guide wire 26 inserted into the first lumen 18 from the distal side through the insertion path 32.

The outer shaft 24 surrounds the inner shaft 20 inserted into the second lumen 22. The inner diameter of the second lumen 22 is set relatively greater when compared to the outer diameter of the inner shaft 20. In a state of disposing the inner shaft 20 in the second lumen 22, a gap 34 capable of allowing distributing of the fluid between the outer surface of the inner shaft 20 and the inner surface of the outer shaft 24 is formed. In addition, the length of the outer shaft 24 in the axial direction is formed shorter than that of the inner shaft 20. Accordingly, the inner shaft 20 extends from both the end portions (distal end and proximal end) of the outer shaft 24 in the axial direction.

The distal end of the outer shaft 24 is welded and held to the side surface of the inner shaft 20 through the first sealing member 28. The first sealing member 28 coaxially supports the inner shaft 20 and the outer shaft 24 in the distal side of the shaft 14.

Further, a plurality of holes 35 (ejection holes) communicating with the second lumen 22 are formed in a position nearby to where the first sealing member 28 of the outer shaft 24 is welded. The holes 35 are spirally punched (spiral cut) into a wall portion of the outer shaft 24 by a drill or the like. The holes 35 eject a predetermined fluid (for example, a priming liquid) supplied through the second lumen 22 into a space portion 42 by allowing inside (space portion 42) of a sheath 36 described below and the second lumen 22 to communicate with each other.

It is preferable that a material constituting the shaft 14 (the inner shaft 20 and the outer shaft 24) be selected from materials capable of obtaining kink resistance and followability suitable for the relationship with a living body lumen (a blood vessel or the like) serving as a target for insertion. Examples of the constituent materials of the shaft 14 may include polyolefins such as a polyethylene, a polypropylene, or an ethylene-vinyl acetate copolymer; polyesters such as a polyamide, a polyethylene terephthalate, or a polybutylene terephthalate; a fluorine-based resin such as a polyvinyl chloride, a polyurethane, a polystyrene-based resin, or a polytetrafluoroethylene; a resin having various flexibilities such as a polyimide; and a synthetic polymer organic material such as a silicone rubber, a polyamide elastomer, a polyester elastomer, a polyvinyl chloride elastomer, or a polyurethane-based elastomer. Note that the inner shaft 20 and the outer shaft 24 may be formed of the same materials or different materials from each other to have different elasticity.

The side surface of the shaft 14 is externally covered by the sheath 36 capable of accommodating the stent graft 16. The sheath 36 is formed of an elongate tubular member extending from behind of the nose cone 30 to the periphery of the distal side of the hub 10, and configured so as to be freely movable back and forth with respect to the shaft 14. The sheath 36 can be formed of the above materials mentioned in the section of the shaft 14.

A sheath hub 38 which can be operated by an operator is attached to the proximal portion of the sheath 36. A second sealing member 40 is provided in inside of the sheath hub 38 and seals the outer surface of the shaft 14. The second sealing member 40 has a function of allowing gas (air) to pass through and blocking the distribution of a fluid.

The space portion 42 extending in the axial direction and having an inner diameter greater than the outer diameter of the outer shaft 24 is formed in the sheath 36. The space portion 42 on the distal side of the sheath 36 accommodates the stent graft 16 in a contracted state. The sheath 36 wholly moves back and forth by operating the sheath hub 38 to be moved back and forth by operation of an operator, and consequently the stent graft 16 is exposed from the space portion 42.

The stent graft 16 is arranged at a position of surrounding the distal side of the outer shaft 24 and the lateral side of the inner shaft 20. In regard to the stent graft 16, a known stent graft in the related art can be applied thereto. Note that a self-expanding type of stent graft which can be deformed from a contracted state to an expanded state by an elastic support (stent) is applied as the stent graft 16 according to the present embodiment, but a balloon-expanding type of stent graft which becomes in the expanded state by expanding a balloon (or other devices) in inside of the stent graft may be also applied. The stent graft 16 is brought into close contact with the inner surface of a living body lumen and then indwelled therein by being exposed from the sheath 36 and being expanded.

In addition, an implant delivered by the catheter 12 may have various configurations in addition to the stent graft 16. For example, as the implant, an artificial valve substituting a valve of a heart or an artery, or an insert (coil member) supplied to an aneurysm is exemplified. In short, a target to be delivered by the catheter 12 is not particularly limited in the present invention.

Fig. 2A is a cross-sectional view taken along line IIA-IIA of the hub 10 of Fig. 1, Fig. 2B is a cross-sectional view taken along line IIB-IIB of Fig. 1, Fig. 2C is a cross-sectional view taken along line IIC-IIC of Fig. 1, Fig. 2D is a cross-sectional view taken along line IID-IID of Fig. 1, and Fig. 2E is a cross-sectional view taken along line IIE-IIE of Fig. 1.

As shown in Figs. 1 to 2E, the hub 10 connected to the proximal side of the shaft 14 is formed in a cylindrical shape. In addition, the hub 10 is formed with a larger diameter than that of the shaft 14 for easy gripping by an operator. The operator activates the distal side of the shaft 14 inserted into a living body lumen by gripping the hub 10 and performing a predetermined operation (back and forth operation or rotating operation).

The hub 10 is configured to have sufficient rigidity compared to the shaft 14 having flexibility. Examples of the materials constituting the hub 10 are not particularly limited, but may include known materials as medical grade materials, for example, polymer materials such as nylon, a polyacrylamide, a polycarbonate, a polyethylene, a polyformaldehyde, a polymethyl methacrylate, a polypropylene, a polytetrafluoroethylene, a polytrifluorochloroethylene, a polyvinyl chloride, a polyurethane, a polyether block amide, and an elastomer organosilicon polymer, or other resin materials, or metal materials (for example, stainless steel and nitinol).

Further, the inside of the hub 10 is formed in a hollow shape such that the proximal side of the shaft 14 is fit and held (hereinafter, referred to as a hollow portion 44). The hollow portion 44 is formed such that the hollow portion penetrates the inside of the hub 10 in the axial direction, and is configured by a holding unit 45 (a first holding unit 46 and a second holding unit 48) which holds the shaft 14, a distribution path 50, and a branch path 52.

The holding unit 45 includes a first holding unit 46 and a second holding unit 48 so as to separately hold the inner shaft 20 and the outer shaft 24 configured as a dual lumen. The first holding unit 46 is a region projected in an axial center direction to have an inner diameter approximately coinciding with the outer diameter of the inner shaft 20 (see Fig. 2D) in an approximately intermediate portion of the hollow portion 44 extending in the axial direction. The outer surface of the inner shaft 20 inserted from the distal side of the hollow portion 44 comes into close contact with an inner shaft fitting hole 54 configured by the inner surface of the first holding unit 46. Consequently, the inner shaft 20 is fixed and held by the first holding unit 46.

The second holding unit 48 is a region formed on a more distal side than the first holding unit 46 and having an inner diameter approximately coinciding with the outer diameter of the outer shaft 24 (see Fig. 2A). The outer surface of the outer shaft 24 inserted from the distal side of the hollow portion 44 comes into close contact with the outer shaft fitting hole 56 configured by the inner surface of the second holding unit 48. Consequently, the outer shaft 24 is fixed and held by the second holding unit 48.

Note that each fitting hole (inner shaft fitting holes 54 and outer shaft fitting holes 56) and adhesive supplying holes 58 communicating with the side surface of the hub 10 are formed in plural in one side portion (lower side in Fig. 1) of the first and second holding units 46 and 48. An adhesive is injected into the adhesive supplying hole 58 from the outside and the adhesive is introduced to the fitting holes 54 and 56 through the adhesive supplying hole 58. Due to the adhesion of this adhesive, the inner shaft 20 and the first holding unit 46, and the outer shaft 24 and the second holding unit 48 are respectively strongly fixed to each other.

The distribution path 50 is formed to have a relatively larger inner diameter, extends from the first holding unit 46 to the proximal end of the hub 10, and communicates with a proximal end opening portion 60 on the proximal side of the hub 10. The guide wire 26 or a fluid is inserted or introduced into the hub 10 from the proximal end opening portion 60. The distal side of the distribution path 50 is formed in a taper shape reducing in diameter toward the inner shaft fitting hole 54, and can easily introduce the guide wire 26 or the fluid into the first lumen 18 of the inner shaft 20 fixed to the inner shaft fitting hole 54.

A groove portion 62 extending in the axial direction from the distal side of the distribution path 50 to the intermediate portion is provided on the inner wall of the hub 10 constituting the distribution path 50 (see Fig. 2E). A plurality of groove portions 62 are provided in the circumferential direction of the distribution path 50 corresponding to the branch path 52 (side path 64). The groove portion 62 stabilizes the flow of the fluid flowing in the distribution path 50 in the axial direction and allows the fluid to smoothly flow into the branch path 52.

The branch path 52 communicates the distribution path 50 with the second lumen 22 of the outer shaft 24 fixed into the second holding unit 48, and has a function of guiding the fluid flowing in the distribution path 50 to the second lumen 22. The branch path 52 is configured by a plurality of side paths 64 extending in the axial direction on the lateral side of the first holding unit 46 and a confluent path 66 in which the plurality of side paths 64 join together between the first holding unit 46 and the second holding unit 48.

The side path 64 includes a proximal end communicating port 68 (opening portion) opening to the distribution path 50 side, and is formed to extend in parallel with the inner shaft fitting hole 54. As shown in Fig. 2D, the plurality (11 in Fig. 2D) of the side paths 64 are provided along the circumferential direction of the first holding unit 46 at equivalent intervals. Note that since the adhesive supplying hole 58 is provided on the lower side of the first holding unit 46 in Fig. 2D, the side path 64 is not formed and the gap between two adjacent side paths 64 is formed to be wider than another.

Each side path 64 is formed to have a long and narrow approximately isosceles triangular shape with the center axis side of the hub 10 as the apex 64a when viewed from a cross-section and extends in the axial direction. Accordingly, the proximal end communicating port 68 of the side paths 64 forms an approximate isosceles triangular shape, and the shape is different from the cross-sectional shape (circle) of the distal portion of the guide wire 26 (see Fig. 4B). Particularly, a dimension X of a side 64b facing the apex 64a of the proximal end communicating port 68 (side path 64) is set smaller than an outer diameter φG of the distal portion of the guide wire 26, and the opening area of the proximal end communicating port 68 is set smaller than the cross-sectional area of the distal portion of the guide wire 26. Therefore, the proximal end communicating port 68 can securely prevent the distal portion of the guide wire 26 from entering the side path 64.

Note that a wrong entry prevention means for the guide wire 26 by the branch path 52 (side path 64) is not limited to the shape of the above proximal end communicating port 68, and may have various configurations. For example, the shape of the proximal communicating port 68 is not limited to a triangle, and may be circular or another polygonal shape. Further, in a case where the opening area of the proximal end communicating port 68 is set larger than the cross-sectional area of the distal portion of the guide wire 26, a net or a lattice which is finer than the cross-sectional area of the distal portion of the guide wire 26, or a sealing member capable of transmitting a liquid may be provided in the proximal end communicating port 68.

Returning to Fig. 1, the distal side of the side path 64 communicates with the confluent path 66. Specifically, the confluent path 66 positioned on a more distal side than the first holding unit 46 is formed with a dimension of covering (overlapping) the apex 64a of the side path 64 extending on the lateral side and with an inner diameter larger than that of the inner shaft fitting hole 54 (see Fig. 2C). In other words, the side path 64 extending in the axial direction communicates with the confluent path 66 by the apex 64a being excavated along with the inner diameter of the confluent path 66 in a portion overlapping with the confluent path 66.

The confluent path 66 is formed as a space surrounding the outer side surface of the inner shaft 20, and communicates with a plurality of the side paths 64 formed in the circumferential direction. Due to this, the fluids guided by the side path 64 temporarily join together. That is, the confluent path 66 can make separate fluids whose flow velocities become faster by flowing in the side path 64 uniform in the confluent path 66.

The distal side of the confluent path 66 communicates with the second lumen 22 of the outer shaft 24 fixed and held by the second holding unit 48 (see Fig. 2B). Accordingly, the branch path 52 can lead the fluid flowing in the distribution path 50 to the second lumen 22 through the side path 64 and the confluent path 66.

The catheter 12 including the hub 10 according to the first embodiment and the hub 10 are basically configured as described above, and the action and the effects of the catheter will be described hereinafter.

The catheter 12 according to the first embodiment is used when the stent graft 16 is delivered and indwelled in inside of a living body through a living body lumen. When the catheter is used, the priming operation is performed to remove the air in the catheter 12 as described above. In the priming operation, a fluid is introduced into the lumen (the first and second lumens 18 and 22) of the catheter 12 and the air present in the lumen is discharged. As the fluid used for the priming operation, a mixture (hereinafter, referred to as a priming liquid) of a sterilized physiological salt solution and an X-ray contrast agent, for example, in a ratio of 1:1 can be used.

Fig. 3 is a partial cross-sectional side view illustrating flow of a fluid at the time of a priming operation in the catheter 12 of Fig. 1. In a case where the priming operation is performed, a syringe (not illustrated) with the priming liquid filled thereinto is connected to the proximal side of the hub 10. The syringe is connected to the proximal end opening portion 60 communicating with the distribution path 50, and the priming liquid is supplied to the hub 10 (distribution path 50) by pressing of a plunger (not illustrated) of the syringe by an operator.

The priming liquid flowing in the distribution path 50 moves to the distal side of the distribution path 50 and a part thereof (the liquid mainly on the central side) is guided to flow into the first lumen 18 by a tapered surface of the distal side. Further, another portion of the priming liquid (the liquid mainly on the inner wall side) is guided along with the groove portion 62 of the distribution path 50 and flows into the plurality of side paths 64 (branch path 52).

The priming liquid flowing in the first lumen 18 is passed through the inner shaft 20 to be led to the distal side of the catheter 12. In addition, the priming liquid is ejected from the distal side of the catheter 12 after the liquid flows into the insertion path 32 of the nose cone 30. That is, the air present in the first lumen 18 is extracted by the priming liquid and the first lumen 18 is filled with the priming liquid.

On the other hand, the priming liquids flowing in the plurality of side paths 64 move to the distal side of the side paths 64 along the respective side paths 64, and temporarily join together in the confluent path 66 communicating with the distal side. Subsequently, the priming liquid flows into the second lumen 22 from the confluent path 66. The priming liquid flowing in the second lumen 22 is passed through the outer shaft 24 to be led to the distal side of the catheter 12. Further, the priming liquid flows out from a plurality of holes 35 formed on the side surface of the distal side of the outer shaft 24 and is supplied to the space portion 42 of the sheath 36.

The priming liquid flowing in the sheath 36 moves toward the stent graft 16 in which a part of the priming liquid is arranged on the distal side of the outer shaft 24, and pushes out the air in an accommodation space (the distal side of the space portion 42) of the stent graft 16, and is discharged from the gap between the nose cone 30 and the sheath 36. Another part of the priming liquid moves to the proximal side of the space portion 42, and discharges the air on the proximal side of the space portion 42 by pushing out the air from the second sealing member 40. Accordingly, the space portion 42 and the second lumen 22 in the sheath 36 are filled with the priming liquid.

As described above, in the catheter 12 according to the first embodiment, since the branch path 52 communicates the second lumen 22 with the distribution path 50 and the distribution of the priming liquid is possible, the priming liquid supplied to the distribution path 50 is allowed to be distributed through the first lumen 18 and the second lumen 22 through the branch path 52. Accordingly, the priming liquid can be supplied to the first and second lumens 18 and 22 at the same time, so the priming operation can be performed efficiently.

In this case, since the priming liquid can be distributed through the plurality of side paths 64, a sufficient amount of the priming liquid to be supplied can be allowed to flow into the second lumen 22. Further, in the priming operation, the priming liquid is ejected from the holes 35 of the second shaft 24, and can be supplied even to the stent graft 16 accommodated in the sheath 36.

Fig. 4A is a partial cross-sectional side view illustrating a state at the time of inserting the guide wire 26 in the catheter 12 of Fig. 1, and Fig. 4B is an explanatory view illustrating a relationship between the proximal end communicating port 68 of the side path 64 and the guide wire 26. In a case where the guide wire 26 is inserted into the catheter 12, the guide wire 26 is inserted from the proximal side (proximal opening portion 60) of the hub 10. In addition, as shown in Fig. 4A, the guide wire is inserted into the shaft 14 (inner shaft 20) along with the first lumen 18 and is sent out to the outside from the nose cone 30 of the distal side.

Here, the distal portion of the guide wire 26 is formed to be bent in many cases as shown in Fig. 4A. In a case where such a guide wire 26 is inserted into the hub 10, the distal portion of the guide wire 26 becomes closer to the inner wall side of the distribution path 50. Therefore, the distal portion of the guide wire 26 faces the side path 64 (proximal end communicating port 68) provided around the first lumen 18 in many cases.

In regard to the guide wire 26, as shown in Fig. 4B, the catheter 12 according to the first embodiment is formed such that the shape of the opening of the proximal end communicating port 68 blocks the entry of the distal portion of the guide wire 26. That is, the opening area of the proximal end communicating port 68 is smaller than the cross-sectional area of the distal portion of the guide wire 26. Consequently, since the side path 64 (proximal end communicating port 68) blocks the entry of the guide wire 26 even when the distal portion of the guide wire 26 faces the proximal end communicating port 68, wrong entry of the guide wire 26 with respect to the second lumen 22 can be inhibited.

As a result, deformation or damage of the guide wire 26 can be prevented. In addition, since a surface on which the proximal end communicating port 68 is formed is inclined along with the tapered surface of the distribution path 50, the distal portion of the guide wire 26 can be easily inserted into the first lumen 18 and the inserting operation of the wire can be performed in a short period of time.

### [First modification example]

Fig. 5A is a partial cross-sectional view illustrating a hub 10A of a catheter 12A according to a first modification example, Fig. 5B is a cross-sectional view taken along line VB-VB of Fig. 5A, Fig. 5C is a cross-sectional view taken along line VC-VC of Fig. 5A, and Fig. 5D is a cross-sectional view taken along line VD-VD of Fig. 5A. Note that a configuration having the same configuration or the same function as that of the catheter 12 of the first embodiment in description of modification examples and embodiments below is denoted by the same reference symbols and the detailed description thereof will not be repeated.

The catheter 12A according to the first modification example is different from the catheter 12 according to the first embodiment in terms that a plurality of lumens (first and second lumens 70 and 72) formed in a shaft 14a are vertically formed without being arranged on the same axis. That is, shafts, which are vertically different, of the first and second lumens 70 and 72 in the catheter 12A extend in parallel as they are and are respectively opened on the distal side of the shaft 14a.

In the hub 10A connected to the proximal side of the catheter 12A, a holding unit 74 holding the shaft 14a, a distribution path 50, and a branch path 52a are formed corresponding to the first and second lumens 70 and 72 of the shaft 14a. A holding unit 74 extends to the intermediate portion of the hub 10 in the axial direction, and fits and holds the proximal side of the shaft 14a.

The distribution path 50 includes a shared space portion 76 having a relatively large inner diameter and communicating with a proximal end opening portion 60, and a lumen channel 78 which overlaps with the shared space portion 76, has a relatively small diameter and communicates with the first lumen 70. The distribution path 50 guides a guide wire 26 or a predetermined fluid (for example, a priming liquid) to the first lumen 70 through the shared space portion 76 and the lumen channel 78.

The branch path 52a formed to have a diameter finer than the lumen channel 78 can distribute a fluid flowing in the distribution path 50 (shared space portion 76). Specifically, a plurality (four in Fig. 5C) of the branch paths 52a are formed in an area facing the second lumen 72, and include a proximal end communicating port 80 communicating with the shared space portion 76 and a distal end communicating port 82 facing the second lumen 72. Therefore, the branch path 52a can allow the priming liquid to easily flow into the second lumen 72 of the shaft 14a.

In other words, as shown in the example of the catheter 12A according to the first modification example, even when the shaft 14a includes the first and second lumens 70 and 72 formed of shafts different from each other, the priming liquid can be supplied to the first and second lumens 70 and 72 at the same time in the priming operation or the like. Further, since the opening area of the proximal end communicating port 80 is formed sufficiently small, the entry of the guide wire 26 into the second lumen 72 is prevented and the guide wire 26 can be inserted into the first lumen 70.

### [Second embodiment]

Fig. 6A is a perspective view illustrating a main part of a hub 110 of a catheter 112 according to a second embodiment, Fig. 6B is a cross-sectional side view of the hub 110 of Fig. 6A, and Fig. 6C is an explanatory view illustrating the hub 110 of Fig. 6A when being visually checked from a proximal end direction. Fig. 7A is an exploded perspective view of the hub 110 of Fig. 6A, Fig. 7B is a cross-sectional side view of a first housing 114 of Fig. 7A, and Fig. 7C is a cross-sectional side view of a second housing 116 of Fig. 7A.

The catheter 112 according to the second embodiment is different from the hubs 10 and 10A of the first embodiment and the first modification example in terms that the hub 110 connected to the proximal side of a shaft 14 is formed of two members (the first and second housings 114 and 116), and is detachably formed in the axial direction and formed to be relatively rotatable in the circumferential direction. In this case, a hollow portion 44a of the first housing 114 is formed to have a second holding unit 48 and a hollow portion 44b of the second housing 116 is formed to have a first holding unit 46.

The first housing 114 includes a main body portion 118 formed in a cylindrical shape and an insertion unit 120 extending in the proximal end direction from the proximal surface of the main body portion 118. An outer shaft fitting hole 56 (second holding unit 48) penetrating in the axial direction is formed in inside of the main body portion 118.

The insertion unit 120 is formed in an arc-shape in the cross section (approximately semicircular shape whose central portion is notched), extends in the axial direction, and is inserted into a confluent path 66 formed in the second housing 116 (see Fig. 7A). The length of the insertion unit 120 in the axial direction coincides with the depth of the confluent path 66 in the axial direction, and a distal end surface 120a is attached to an end surface 66a (bottom surface) of the confluent path 66 in a state in which the insertion unit 120 is inserted into the confluent path 66.

Further, the insertion unit 120 is formed in a shape whose arc-shaped outer surface approximately coincides with the inner surface of the confluent path 66 of the second housing 116, and the arc-shaped inner surface side is formed to be flush with the inner surface of an inner shaft fitting hole 54 of the second housing 116. Accordingly, the outer surface of the insertion unit 120 is brought into close contact with the inner surface of the second housing 116 with sufficient friction force and the first housing 114 and the second housing 116 are connected to each other by inserting the insertion unit 120 into the second housing 116 (confluent path 66).

On the other hand, the second housing 116 is formed in a cylindrical shape to be flush with the first housing 114, and the confluent path 66, a first holding unit 46, a side path 64, and a distribution path 50 are formed in a hollow portion 44b penetrating in the axial direction in this order from the distal side.

The insertion unit 120 of the first housing 114 is inserted into the confluent path 66 of the distal side of the second housing 116. Since the insertion unit 120 is formed in an arc-shape in the cross section (approximately semicircular shape) described above, the confluent path 66 has an approximate half space (a movable space 122: a space on the side with the insertion unit 120 not being inserted) in a state of the insertion unit 120 being inserted (see Fig. 8A). In the movable space 122, a forming position in the confluent path 66 is changed according to the insertion position of the insertion unit 120. In a case where the movable space 122 communicates with the side path 64, a fluid (priming liquid) flowing from the side path 64 flows into a second lumen 22 of the outer shaft 24 held by the first housing 114 through the movable space 122.

The side path 64 formed on the lateral side of the first holding unit 46 is different from that of the first embodiment, and a plurality (six) of the side paths are formed along the circumferential direction of a half side (approximately lower side in Fig. 6C) of the second housing 116 as shown in Fig. 6C. The side path 64 includes a distal end communicating port 124 opened to the confluent path 66. Therefore, when the second housing 116 is visually checked from the confluent path 66 side, it is confirmed that a plurality of the distal end communicating ports 124 are arranged in an approximate arc-shape (approximately semicircular shape) along the circumferential direction of the half side. In addition, the cross-sectional area of the side path 64 is formed slightly larger than that of the side path 64 of the first embodiment, and is configured such that the amount of a fluid flowing into the second lumen 22 is large and not reduced even when the number of side paths 64 formed is small.

Fig. 8A is a partial cross-sectional side view illustrating flow of a fluid supplied to the hub 110 of Fig. 6A, and Fig. 8B is a partial cross-sectional side view illustrating flow of a fluid in a state in which the first housing 114 and the second housing 116 of the hub 110 of Fig. 8A are relatively rotated by 180° with respect to each other.

The hub 110 according to the second embodiment can selectively change the state of supplying (including adjustment of the amount to be supplied in addition to supplying or blocking) a fluid with respect to the second lumen 22 by the connection state of the first housing 114 and the second housing 116. That is, since the hub 110 is configured such that the first and second housings 114 and 116 are separate from each other, the positional relationship between the insertion unit 120 and the side path 64 can be changed, and communication, closure (blocking), and the like of the branch path 52 can be switched.

As shown in Fig. 8A, the distal end communicating port 124 communicates with the movable space 122 of the confluent path 66 in a state in which the distal surface 120a of the insertion unit 120 does not face the side path 64 (distal end communicating port 124). Accordingly, a fluid (priming liquid) flows in through the side path 64, and then the fluid flows into the second lumen 22 through the movable space 122. In other words, since the priming liquid flowing in the distribution path 50 distributes through both the first and second lumens 18 and 22, the priming operation can be efficiently performed.

As shown in Fig. 8B, the distal surface 120a of the insertion unit 120 can be put in a connection state corresponding to the side path 64 (distal end communicating port 124) from the connection of Fig. 8A by relatively rotating the first housing 114 with respect to the second housing 116. In the state in which the distal surface 120a corresponds to the distal end communicating port 124, the branch path 52 is blocked by the insertion unit 120. Therefore, the fluid does not flow into the second lumen 22, but only flows into the first lumen 18.

In the connection state shown in Fig. 8B, for example, advantageous effects can be obtained when a contrast agent (a liquid for X-ray fluoroscopic image photographing) is supplied through the first lumen 18 and the distal side of the catheter 112 is read. That is, when the contrast agent is supplied to the stent graft 16 (see Fig. 1) accommodated in the distal side of the catheter 12 through the second lumen 22 in a case where the contrast agent is supplied to both the first and second lumens 18 and 22, the stent graft 16 becomes blurred. In contrast, when the contrast agent is supplied only to the first lumen 18 without supplying the contrast agent to the stent graft 16 in the connection state of Fig. 8, the contrast agent can be ejected from the distal end (nose cone 30) of the catheter 112. As a result, the vicinity of the distal side of the catheter 112 can be read with high precision without the stent graft 16 being blurred.

As described above, according to the catheter 112 including the hub 110 according to the second embodiment and the hub 110, since the hub 110 can be separated into the first and second housings 114 and 116, the inner portion of the hub 110 can be accessed from the separated portion and cleaning the inside of the hub 110 or the like can be easily performed. Further, a fluid can be selectively supplied to the second lumen 22 by blocking the communication between the second lumen 22 and the distribution path 50 along with the rotation of the first housing 114 or the second housing 116. Therefore, the usability of the catheter 112 is further improved.

### [Second modification example]

Fig. 9A is a partial cross-sectional view illustrating a hub 110A of a catheter 112A according to a second modification example, and Fig. 9B is a partial cross-sectional side view illustrating a state in which a first housing 114a and a second housing 116a of the hub 110A of Fig. 9A are relatively rotated by 180° with respect to each other. The hub 110A of the catheter 112A according to the second modification example includes a second holding unit 48, a confluent path 66, a first holding unit 46, and a side path 64 in a first housing 114a, and is different from the hub 110 of the catheter 112 according to the second embodiment in terms of including a distribution path 50 in a second housing 116a.

An opening portion of a first lumen 18 and a proximal end communicating port 68 of the side path 64 are formed in the proximal side of the first housing 114a, and a proximal end projecting portion 130 fixing and holding the second housing 116a is formed such that the proximal end projecting portion 130 surrounds the periphery.

On the other hand, the second housing 116a includes a distribution path 50 thereinside and an insertion unit 132 is formed such that the insertion unit surrounds the distribution path 50 and can be fitted and inserted into the proximal end projecting portion 130. An arc-shaped (approximately semicircular shape) closing unit 134 which can be attached to the proximal end communicating port 68 is provided on the distal side of the insertion unit 132.

In a state in which the closing unit 134 does not face the proximal end communicating port 68 (in a state in which the closing unit 134 is positioned on the upper side of the first lumen 18 in Fig. 9A), the hub 110A according to the second modification example can distribute a fluid (for example, a priming liquid) to the first lumen 18 and a second lumen 22. On the other hand, in a state in which the closing unit 134 faces the proximal end communicating port 68 (in a state in which the closing unit 134 is positioned in the lower side of the first lumen 18 in Fig. 9B), a fluid (for example, a contrast agent) can be distributed only through the first lumen 18 because a branch path 52 is blocked.

That is, as shown in the hubs 110 and 110A, the separated sites and the rotatable sites of the first and second housings 114, 114a, 116, and 116a can be freely designed without particular limitation.

In the above description, the present invention has been described with reference to preferred embodiments, but the present invention is not limited thereto, and various modifications are obviously possible within a range not departing from the scope of the present invention. For example, the catheters 12, 12A, 112, and 112A which can be provided with the hubs 10, 10A, 110, and 110A of the present invention are not particularly limited to catheters delivering an implant and can be applied to various catheters which can be inserted into a living body.

In addition, the lumens formed in the shafts 14 and 14a of the catheters 12, 12A, 112, and 112A are not particularly limited to these two, which are the first lumen 18 or 70 and the second lumen 22 or 72, and three or more lumens may be provided. In this case, a fluid may be supplied by increasing the number of the branch paths 52 according to the number of lumens to be arranged, or a fluid may be supplied to a plurality of lumens from one branch path 52.

## Claims

1. A hub (10, 10A, 110, 110A) which is connected to a shaft (14, 14a) including a lumen (18, 70) in which a wire is capable of being inserted, and a cavity portion (22, 72) separately provided from the lumen (18, 70), comprising:
a holding unit (45, 74) which holds the shaft (14, 14a) ;
a distribution path (50) which communicates with the lumen (18, 70) of the shaft (14, 14a) held by the holding unit (45, 74) and is capable of allowing inserting of the wire and distributing a fluid; and
a branch path (52, 52a) which communicates the cavity portion (22, 72) of the shaft (14, 14a) held by the holding unit (45, 74) with the distribution path (50), is capable of distributing the fluid, and blocks the insertion of the wire.

2. The hub (10, 110, 110A) according to claim 1,
wherein the hub (10, 110, 110A) is configured to be connected to the shaft (14) which includes a first shaft (20) having the lumen (18) and a second shaft (24) having the cavity portion (22) and capable of accommodating the first shaft (20) in the cavity portion (22),
the holding unit (45) includes a first holding unit (46) which holds the first shaft (20) and a second holding unit (48) which is provided on a more distal side than the first holding unit (46) and holds the second shaft (24),
the distribution path (50) is provided on the proximal side of the first holding unit (46), and
the branch path (52) includes a side path (64) which communicates with the distribution path (50) and extends in the axial direction on the lateral side of the first holding unit (46).

3. The hub (10, 110, 110A) according to claim 2,
wherein the side path (64) is configured such that an opening portion (68) opened to the distribution path (50) is formed in a shape capable of blocking the entry of the distal portion of the wire.

4. The hub (10, 110, 110A) according to claim 3,
wherein a plurality of the side paths (64) are provided along the circumferential direction of the first holding unit (46).

5. The hub (10, 110, 110A) according to claim 2,
wherein a groove portion (62) capable of leading the fluid into the side path (64) is provided on the inner surface constituting the distribution path (50).

6. The hub (110, 110A) according to claim 2,
wherein the hub (110, 110A) is configured by a first housing (114, 114a) and a second housing (116, 116a) which are separated from each other in the axial direction.

7. The hub (110, 110A) according to claim 2,
wherein the hub (110, 110A) is configured such that a first housing (114, 114a) and a second housing (116, 116a) which are separated from each other are rotatably connected to each other, and
the branch path (52) is configured such that the communication between the cavity portion (22) and the distribution path (50) is blocked along with relative rotation of the first housing (114, 114a) with respect to the second housing (116, 116a).

8. A catheter (12, 112, 112A) including the hub (10, 110, 110A) according to claim 2 and the shaft (14, 14a),
wherein the hub (10, 110, 110A) is configured such that a space portion (42) capable of accommodating an implant implanted in a living body is provided on an outer surface of the distal side of the first shaft (20), and
the second shaft (24) is capable of supplying the fluid which is guided into the distal side through the cavity portion (22) to the space portion (42).

9. The catheter (12, 112, 112A) including the hub according to claim 8, wherein the hub is configured such that the second shaft (24) includes ejection holes (35) which communicate with the space portion (42) and eject the fluid.
